Europäisches Patentamt

(19) **European Patent Office**

**Office européen des brevets**

(11) Numéro de publication: **0 005 666**
**B1**

(12) **FASCICULE DE BREVET EUROPEEN**

(45) Date de publication du fascicule du brevet: **20.01.82**

(51) Int. Cl.³: **A 61 B 6/02, H 05 G 1/70**

(21) Numéro de dépôt: **79400298.0**

(22) Date de dépôt: **11.05.79**

(54) **Appareil tomographique à gaine unique.**

(30) Priorité: **16.05.78 FR 7814406**

(43) Date de publication de la demande:
**28.11.79 Bulletin 79/24**

(45) Mention de la délivrance du brevet:
**20.01.82 Bulletin 82/3**

(84) Etats contractants désignés:
**DE GB NL**

(56) Documents cités:
**DE - A - 2 708 612**
**FR - A - 2 284 113**
**FR - A - 2 304 321**
**FR - A - 2 341 149**
**FR - A - 2 368 930**
**FR - A - 2 374 885**
**FR - A - 2 385 374**
**FR - A - 2 391 697**
**US - A - 4 126 786**

(73) Titulaire: **COMPAGNIE GENERALE DE RADIOLOGIE**
**13 square Max-Hymans**
**F-75741 PARIS CEDEX 15 (FR)**

(72) Inventeur: **Laffitte, André**
**"THOMSON-CSF" 173, bld Haussmann**
**F-75360 Paris Cedex 08 (FR)**
Inventeur: **Delair, Jacques**
**"THOMSON-CSF" 173, bld Haussmann**
**F-75360 Paris Cedex 08 (FR)**

(74) Mandataire: **Schmolka, Robert et al,**
**"THOMSON-CSF" - SCPI 173. bld Haussmann**
**F-75360 Paris Cedex 08 (FR)**

Courier Press, Leamington Spa, England.

## Appareil tomographique à gaine unique

La présente invention concerne un appareil de tomographie axiale transverse utilisant un ordinateur et comportant une gaine unique porteuse de nombreux tubes à rayons X et d'une pluralité de détecteurs.

Les appareils de tomographie avec ordinateur comportaient à l'origine une source émettant un fin pinceau de rayons X en direction d'une tranche d'un corps à examiner, et un détecteur disposé de façon à recevoir le rayonnement et à mesurer son intensité après qu'il ait traversé le corps. L'ensemble source-détecteur était soumis à un mouvement rectiligne perpendiculaire à la direction du faisceau, puis à une rotation de petit angle autour d'un axe perpendiculaire au plan d'examen, puis, à nouveau à un mouvement rectiligne, et ainsi de suite, jusqu'à ce que cet ensemble ait tourné d'environ 180°.

Il en résultait des temps trop longs pour réaliser des coupes dans les régions d'un organe en mouvement, tel que le coeur, ou un temps inférieur à environ 0,1 S est nécessaire.

Pour atteindre cette performance, il est nécessaire d'éviter ou tout au moins de réduire au minimum les mouvements mécaniques (translation, rotation) des sources des rayons X et des détecteurs.

Dans certains appareils de tomographie, les mouvements de balayage rectilignes ont été supprimés. La source émet alors un faisceau de rayons X en éventail, de grande ouverture dans le plan de coupe, qui traverse le corps et irradie simultanément une pluralité de détecteurs disposés côte à côte dans ce plan.

L'ensemble source-détecteurs n'est plus soumis qu'à un mouvement de rotation autour du corps.

Dans d'autres appareils les mouvements mécaniques de translation de la source sont remplacés par un balayage du faisceau électronique du tube à rayons X sur une cible émettrice de rayons X, représentant la longueur du mouvement de translation.

Mais dans tous ces cas le temps d'acquisition est encore long par suite du mouvement mécanique de rotation du tube à rayons X.

Plusieurs brevets ont préconisé la suppression des mouvements mécaniques de rotation, apparents, du tube à rayons X avec un tube de forme toroïdale de grand diamètre. Cependant, la réalisation de tels tubes est, en l'état actuel de la technique, très difficile et très onéreuse.

Dans la demande de brevet FR—A—2.284.113, on a préconisé l'emploi de plusieurs tubes à faisceau de rayons X en éventail, placés suivant un cercle, au centre duquel doit être placé le corps à examiner. D'autre part plusieurs détecteurs sont placés chaque fois entre deux tubes adjacents, de façon à être irradiés par le tube qui leur est diamétralement opposé. C'est-à-dire que chaque faisceau en éventail produit par un tube, irradie les détecteurs placés entre les deux tubes qui lui font face.

Cependant à l'aide de ce dispositif, il n'est possible d'irradier simultanément un corps de dimension normale, que dans un nombre de directions insuffisant pour une coupe tomographique. En effet, l'ouverture de faisceau en éventail d-un tube est limitée par le nombre des détecteurs correspondants, c'est-à-dire par la distance entre deux tubes. Pour que celle-ci soit suffisante le nombre de tubes doit être faible. Par ailleurs ce document ne décrit pas un arrangement spécial pour maintenir les tubes et les détecteurs.

Pour remédier à cet inconvénients, il a été proposé de faire tourner ce ensemble d'un angle de $2\pi/n$ (n = le nombre de tubes à rayons X) par petites étapes, de manière à multiplier le nombre des directions de mesure d'absorption. Le nombre de tubes étant faible, l'angle de rotation sera grand, et le temps d'acquisition sera encore trop long pour réaliser des coupes d'un organe en mouvement. De plus chaque tube devant être muni d'une gaine protectrice et de systèmes de déclenchement, ce dispositif apparaît comme très encombrant et mal adapté pour une rotation.

L'invention a pour objet un appareil tomographique avec un support spécial pour les éléments émetteurs-récepteurs n'ayant pas tous ces inconvénients et permettant de réaliser des coupes très rapidement.

Dans ce but, l'appareil de l'invention, où les mouvements de translation ont été supprimés comme dans l'état de la technique précitée, comporte une organisation nouvelle des tubes et des détecteurs au moyen d'un support spécial. En effet, les tubes sont placés très près les uns des autres, les intervalles libres portant des détecteurs, ce qui réduit au minimum les mouvements de rotation à effectuer et, qui plus est, facilite ces quelques mouvements de rotation, permettant une tomographie très rapide.

Selon l'invention, pour créer ce support spécial un appareil de tomographie axiale transverse comportant une pluralité de tubes 4 à faisceau de rayons X en éventail, répartis sur un cercle de manière à entourer complètement un corps 5 à examiner placé au centre du cercle, chaque tube 4 étant associé à un dispositif de déclenchement 11 de sa cathode, à des moyens de collimation 12 et à une pluralité de détecteurs 7 de rayons X également répartis sur un cercle de manière à être placés entre les tubes est caractérisé en ce que les tubes 4 sont placés côte à côte à l'intérieur d'une gaîne unique creuse, formée de deux anneaux concentriques 1, 2 et de deux flasques latéraux 3, munie de moyens de refroidissement fluide isolant électriquement, plombée intérieurement et percée de fenêtres 6 de collimation au regard de chaque tube 4 et en ce que les détecteurs (7) sont disposés côte à côte entre le fenêtres sur l'anneau intérieur (1).

La demande de brevet FR—A—2.341.149 décrit une source de rayonnements multiples constituée de plusieurs tubes de Roentgen inclus dans un réservoir. Ceci permet de réaliser une image codée du corps examiné. Chaque tube est relié au même générateur. Tous les tubes sont donc chargés simultanément. Ceci ne permet pas de réaliser un balayage en rotation du corps à examiner propre à reconstruire l'image d'une coupe par la méthode de tomographie classique.

Suivant d'autres caractéristiques de l'invention le gaîne peut également contenir des systèmes de polarisation des cathodes, des circuits de déclenchement de la haute tension des tubes, ainsi que des interfaces isolés de la haute tension assurant une liaison directe entre le circuit de commutation qui permet d'ordonner l'arrêt ou le déclenchement des tubes, et le circuit de déclenchement. On arrive donc ainsi à éviter l'encombrement des dispositifs volumineux d'isolement de la haute tension qui étaient nécessaires dans les appareils à plusieurs tubes précédemment décrits.

D'autres caractéristiques de l'invention ressortiront de la description suivante, donnée à titre d'exemple non limitatif et illustrée par les figures annexées qui représentent:
— la figure 1, une vue schematique de la gaine porteuse de tubes à rayons X d-un appareil tomographique selon l'invention;
— la figure 2, une vue schématique en coupe d'un appareil tomographique selon l'invention;
— la figure 3, un schéma de la disposition des tubes et de leurs systèmes de commande dans la gaine d'un appareil tomographique selon l'invention;
— la figure 4, un schéma d'un tube à rayons X et de son dispositif de déclenchement, équipant un appareil tomographique selon l'invention;
— la figure 5, un dispositif de polarisation et de commande des tubes à rayons X d'un appareil tomographique selon l'invention.

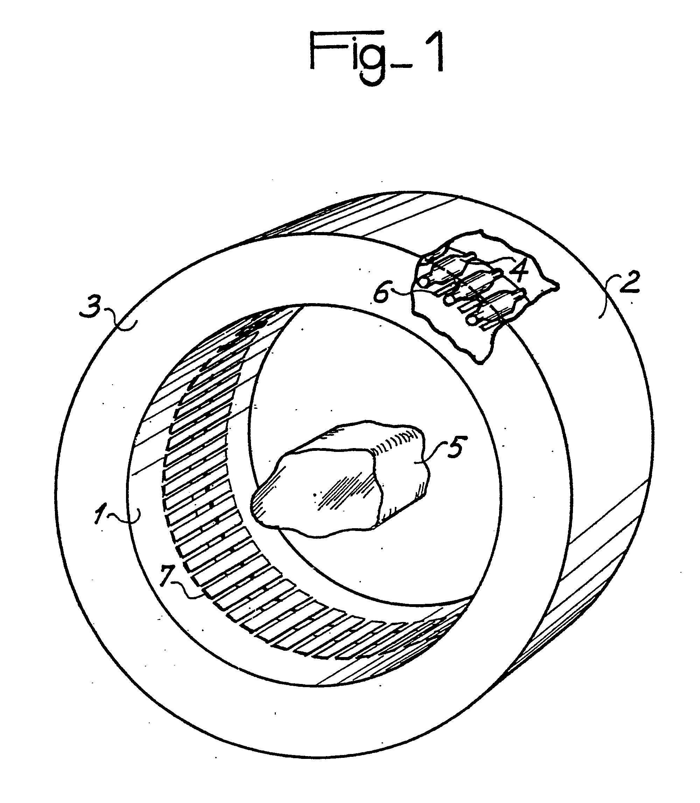

La figure 1 est une vue en perspective de la gaine, porteuse de nombreux tubes à rayons X, d'un appareil tomographique selon l'invention.

Cette gaine comporte un anneau central 1, un anneau extérieur 2, et deux flasques latéraux 3.

Des tubes 4, sont disposés côte à côte à l'intérieur de celle-ci de manière à entourer complètement un corps 5 placé en son centre.

Ces tubes peuvent être par exemple des tubes à anode fixe engendrant des faisceaux de rayons X en éventail de large ouverture.

L'anneau central 1 comporte des fenêtres 6 placées devant chaque tube de manière à permettre le passage du rayonnement provenant de chacun d'eux, et des groupes de détecteurs 7 placés sur sa surface en regard du corps de manière à dégager les fenêtres 6.

Chaque tube 4 peut donc émettre un faisceau de rayons X au travers d'une fenêtre 6 correspondante, irradier le corps 5 et être recueilli, atténué, par un certain nombre de détecteurs correspondant à l'ouverture du faisceau et à la taille du corps.

La gaine, qui peut avoir par exemple un diamètre d'un mètre, peut contenir un nombre relativement important de tubes, car en effet en l'état actuel de la technique des tubes d'une puissance allant jusqu'à 150 KV, ne présentent qu'un encombrement très faible (par exemple: un diamètre de ballon de 5 cm). Dans ces conditions on peut arriver à avoir un angle au centre entre deux tubes c'est-à-dire l'angle entre deux rayons des anneaux 1, 2 passant par le foyer de chacun des deux tubes, inférieur à 10°.

Afin d'accroître encore le nombre de directions de mesure de l'absorption, on peut faire tourner la gaine sur son axe, par petites étapes ou de façon continue d'un angle maximum égal à l'angle au centre entre deux tubes.

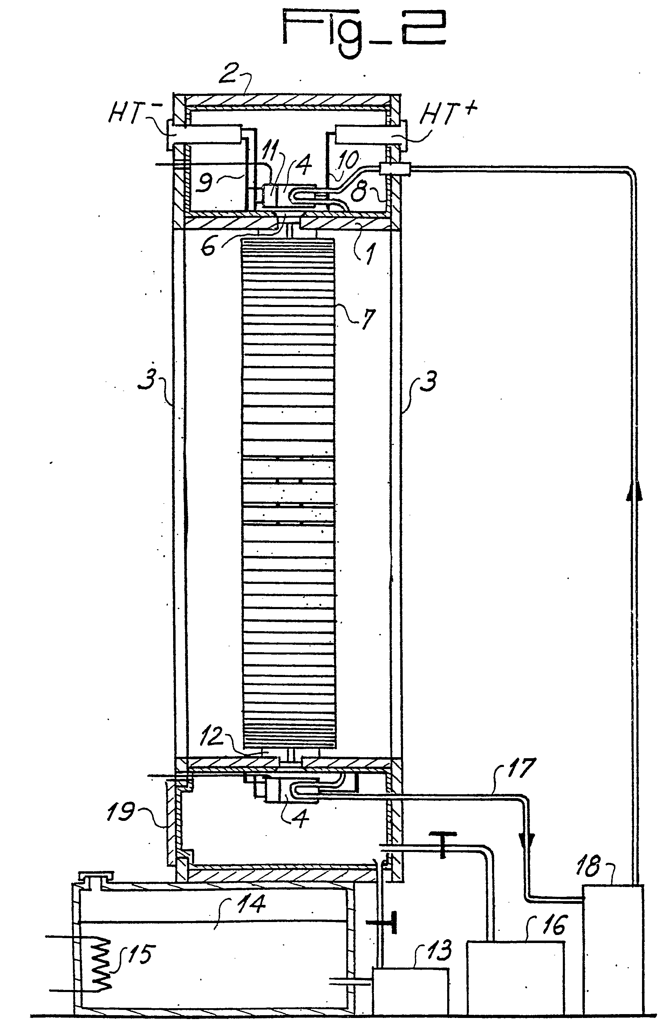

La faible valeur de cet angle, et le fait que tout soit inclus dans une gaine unique comme il le sera décrit à la figure 2, facilitent cette opération et la rendent très rapide.

En effet, la figure 2, représente une vue schématique en coupe d'un appareil tomographique selon l'invention.

L'intérieur de la gaine composée des deux anneaux 1 et 2 et des deux flasques latéraux 3 a sa surface intérieure recouverte d'une couche de plomb 8, à l'exception des surfaces des fenêtres 6.

De plus, devant chacun des tubes, entre les fenêtres et les détecteurs, sont placés des dispositifs de collimation à fente 12.

Tous les tubes 4, sont alimentés en parallèle par des rails latéraux 9 et 10. Les rails 9 sont reliés à un embout HT⁻ fixé sur la gaine, et délivrent aux cathodes des tubes, une tension de chauffage du filament, ainsi que la haute tension négative. Le rail 10 est relié à un second embout HT+ lui aussi fixé sur la gaine, mais susceptible d'être connecté au pôle positif de la haute tension HT+. Tous les tubes ont leurs fils d'alimentation de cathode reliés au rail 9 et d'alimentation d'anode au rail 10.

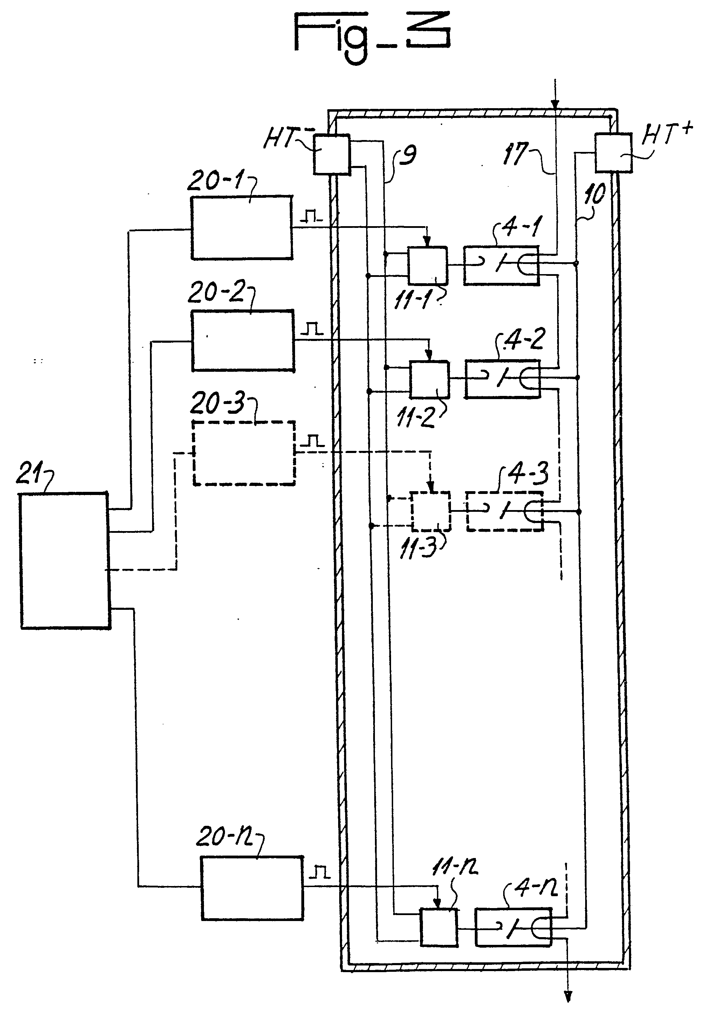

La gaine contient également en 11, less systèmes de polarisation des cathodes, les circuits de déclenchement de la haute tension des tubes, ainsi que des interfaces isolés de la haute tension assurant la liaison entre le circuit de commutation et le circuit de déclenchement. Ces circuits sont détaillés aux figures 3, 4 et 5.

Tout l'intérieur de la gaine est rempli d'huile ou de gaz de manière à l'isoler èlectriquement des tubes.

Dans le cas des l'isolement à l'huile, la gaine est associée à un ensemble pompe à huile 13, réservoir à huile 14 lui-même équipé de moyens de réchauffement, telle qu'une résistance chauffante, pompe à vide 16, permettant ainsi une vidange et un remplissage rapide de l'huile traitée.

La gaine possède des trappes de visite 19 permettant ainsi une maintenance aisée de l'ensemble des tubes et des différents dispositifs qui sont à l'intérieur.

On peut également associer à la gaine un dispositif de refroidissement des anodes, composé d'un circuit 17 permettant une circulation d'huile dans les anodes et d'un refroidisseur d'huile 18.

La figure 3, schématise la disposition des tubes et de leurs systèmes de commande dans la gain d'un appareil tomographique selon l'invention.

Les n tubes 4-1, 4-2,...4-n ont leurs cathodes connectées respectivement aux circuits de déclenchement de la polarisation 11-1, 11-2,... 11-n.

Ces circuits sont connectés d'une part au rail 9 (tension de chauffage — haute tension négative) et d'autre part à des circuits de commutation 20-1, 20-2,... 20-n. Les circuits de commutation sont eux-mêmes reliés à un circuit de commande des commutations 21. Celui-ci permet d'agencer le fonctionnement des tubes à rayons X de manière qu'ils se déclenchent, soit successivement par unité ou par groupe, soit simultanément.

Le déclenchement de chaque tube est directement dû à l'état de sont circuit de déclenchement qui est lui-même commandé par le circuit de commande des commutations.

A la figure 3, tous les tubes à rayons X ont leur anode reliée à un rail 10 lui-même connecté à la borne HT$^+$. De plus un circuit de refroidissement des anodes 17 permet une circulation d'huile refroidie dans les anoders de tous ces tubes.

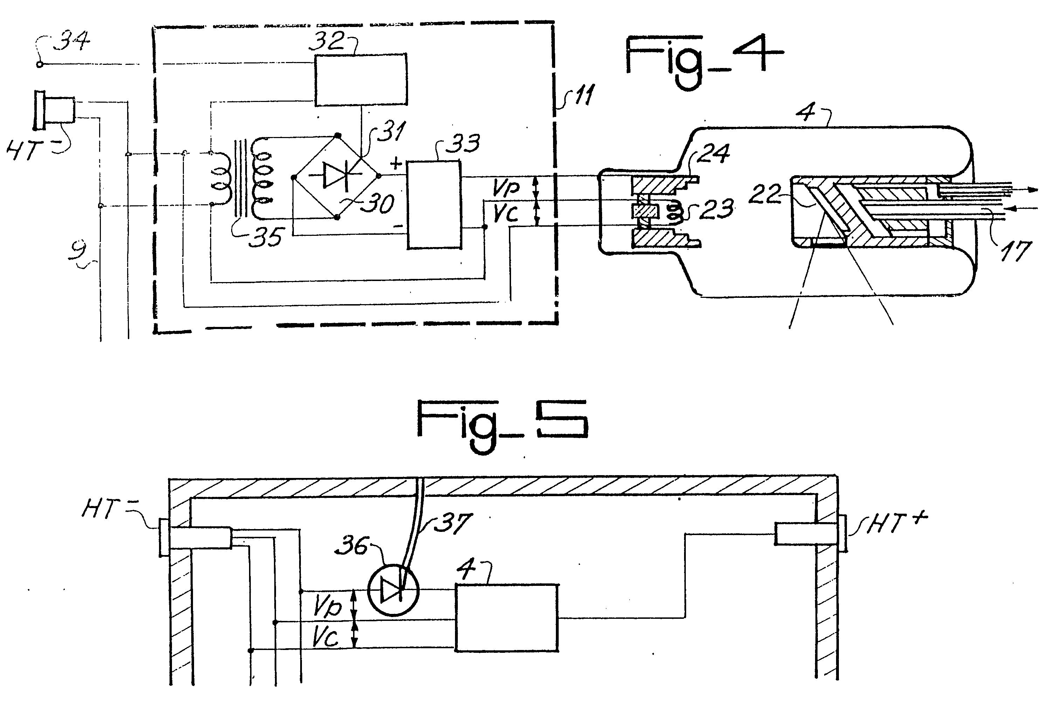

La figure 4 représente un exemple de tube à rayons X et de son système de déclenchement. Le tube 4 est un tube à anode fixe 22, et sa cathode 23 possède un dispositif de polarisation 24. De plus l'anode 22 est creusée de manière à avoir une circulation d'huile grâce au dispositif 17.

Lorsqu'on applique sur le dispositif de polarisation 24 une tension VP, dite de blocage, de l'ordre de −1 KV, on interdit toute émission d'électrons par la cathode. Par contre lorsque cette tension est annulée, la cathode émet des électrons et donc l'anode émet des rayons X.

Car en effet, la cathode est alimentée en permanence par une tension VC, dite de chauffage, de manière à ce que l'émission d'électrons ait lieu immédiatement après l'annulation de la tension de blocage.

Le dispositif de blocage étant relié à la haute tension, et la commande ne pouvant se faire qu'à partir de la basse tension, un isolement est nécessaire.

On utilise, figure 4, un pont de thyristors 30 couplé à la haute tension par l'intermédiaire d'un transformateur 35 et dont les gâchettes reçoivent le signal de commande.

En effet, les gâchettes 31 des thyristors sont connectées à un circuit de mise en forme 32 qui reçoit les impulsions de commande et les transforme en signaux carrés adaptés aux gâchettes des thyristors. Ceux-ci, qui en l'absence de signal de commande étaient bloqués, deviennent conducteur. On a alors une tension aux bornes d'entrée du filtre 33, qui permet d'appliquer au dispositif de polarisation 24, une tension négative par rapport à la cathode, suffisante pour bloquer l'émission d'électrons, et donc l'émission de rayons X.

On peut également utiliser une fibre optique transmettant un signal lumineux vers le système de déclenchement tout en assurant un isolement avec la haute tension.

C'est le cas de la figure 5, où le tube 4, dont l'anode est reliée à HT$^+$ et la cathode polarisable à HT$^-$, possède dans son circuit de polarisation un dispositif d'interruption 36 de la tension de blocage, commandable par fibre optique 37. Ce peut-être par exemple une photo-diode ou encore une cellule photo-électrique.

Dans le cas de cette figure, la tension de polarisation n'est disponible qu'à l'extérieur de la gaine, mais il est tout à fait possible de se ramener au cas de la figure 4. Sur cette figure la tension de polarisation était disponible en sortie du filtre 33 à l'intérieur de la gaine.

Par déclenchement successif des tubes on irradie le corps sur toute sa tranche par un faisceau en éventail d'ouverture assez grande, dans un grand nombre de directions. On peut encore augmenter ce nombre en faisant tourner la gaine par petites étapes ou de façon continue d'un angle égal á l'angle au centre entre deux tubes, qui est, selon l'invention, très petit.

Dans ce cas, le rayonnement X atténué par le ps est reçu par les détecteurs placés sur la gaine porteuse de tubes à rayons X. Ces détecteurs sont disposés côte entre les fenêtres permettant ainsi le passage des rayons X émis par les différents tubes.

Les appareils tomographiques selon l'invention, couplés avec des ordinateurs permettent d'effecteur des coupes dans des régions du corps où il existe des organes en mouvement, tel que le coeur.

## Revendications

1. Appareil de tomographie axiale transverse comportant une pluralité de tubes (4) à faisceau de rayons X en éventail, répartis sur un cercle de manière à entourer complètement un corps (5) à examiner placé au centre du cercle, chaque tube (4) étant associé à un dispositif de déclenchement (11) de sa cathode, à des moyens de collimation (12) et à une pluralité de détecteurs (7) de rayons X également répartis sur un cercle de manière à être placés entre les tubes, caractérisé en ce que les tubes (4) sont placés côte à côte à l'intérieur d'une gaîne unique creuse, formée de deux anneaux concentriques (1, 2) et de deux flasques latéraux (3), munie de moyens de refroidissement fluide isolant électriquement, plombée intérieurement et percée de fenêtres (6) de collimation au regard de chaque tube (4) et en ce que les détecteurs (7) sont disposés côte à côte entre les fenêtres sur l'anneau intérieur (1).

2. Appareil tomographique suivant la revendication 1, caractérisé en ce que les tubes (4) à faisceau de rayons X, sont des tubes à cathode (23) associée à un dispositif de polarisation (24) et à surface anodique (22) fixe et inclinée, produisant en conjonction avec les moyens de collimation (12), un faisceau de rayons X en éventail, plat et de grande ouverture.

3. Appareil tomographique suivant l'une des revendications 1 ou 2, caractérisé en ce que tous les tubes (4) sont munis à l'intérieur de la gaîne de rails, communs (9, 10) d'alimentation en haute tension, de dispositifs de déclenchement (11) et d'un circuit unique de refroidissement (13, 14, 16, 17, 18) par huile de leurs anodes.

4. Appareil tomographique suivant l'une des revendications 1 à 3, caractérisé en ce que tous les systèmes de déclenchement (11) de la cathode sont reliés par un dispositif isolé de la haute tension, tel qu'une fibre optique (37), à des circuits de commutation (20-1,..., 20-n), eux-mêmes reliés à un circuit de commande (21), permettant ainsi un déclenchement très rapide et sélectif des tubes, par exemple l'un après l'autre.

5. Appareil tomographique suivant l'une des revendications 1 à 4, caractérisé en ce que la gaine peut tourner autour du centre du cercle où sont situés les tubes (4) d'un angle égal à l'angle au centre séparant deux tubes successifs.

## Claims

1. Tomographic device for transverse axial tomography, comprising a plurality of tubes (4) producing X-ray beams defining a fan-like configuration, which tubes are distributed on a circle so as to surround completely a body (5) to be examined that is placed in the centre of said circle, each tube (4) being associated to a tripping device (11) for tripping the cathode of said tube, to collimating means (12) and to a plurality of X-ray detectors (7) which are also distributed on a circle so as to be disposed between said tubes, characterized in that tubes (4) are placed side by side within a single hollow envelope constituted by two concentric rings (1, 2) and two lateral flanges (3), said envelope being provided with electrically isolating fluid cooling means, said envelope further being provided with an inner lead layer and with collimating windows (6) facing each of said tubes (4), and in that said detectors (7) are disposed side by side between said windows on the inner ring (1).

2. Tomographic device according to claim 1, characterized in that the X-ray beam producing tubes (4) have each a cathode (23) associated to a polarizing device (24), each tube having further a fixed and inclined anodic surface (22) which produces in combination with said collimating means (12) a flat, widely spread X-ray beam of a fan-like configuration.

3. Tomographic device according to any one of claims 1 or 2, characterized in that all of said tubes (4) are provided, within said envelope, with common high-tension supply bars (9, 10), with tripping devices (11) and with a single cooling oil circuit (13, 14, 16, 17, 18) for cooling the anodes of said tubes.

4. Tomographic device according to any one of claims 1 to 3, characterized in that all the cathode tripping systems (11) are connected, through a device isolated from the high tension, such as an optical fibre (37), to commutation circuits (20-1,..., 20-n) which are connected to a control circuit (21), so as to allow the tubes to be tripped selectively and very quickly, for example one after the other.

5. Tomographic device according to any one of claims 1 to 4, characterized in that said envelope is adapted to rotate about the centre of the circle on which the tubes (4) are disposed, the amplitude of such rotating movement being equal to the central angle separating any two successive tubes.

## Patentansprüche

1. Gerät für axiale Quertomographie mit einer Mehrzahl von Röntgenstrahlenbündel in fächerartiger Anordnung erzeugenden Röhren (4), die derart auf einem Kreis angeordnet sind, dass sie einen in der Mitte dieses Kreises befindlichen Prüfling (5) gänzlich umgeben, wobei jede Röhre (4) mit einer ihre Kathode auslösenden Auslösevorrichtung (11), sowie mit Kollimationsmitteln (12) und einer Mehrzahl von zwischen den Röhren und ebenfalls auf einem Kreis angeordneten Röntgenstrahlendetektoren (7) zusammenwirkt, dadurch gekennzeichnet, dass die Röhren (4) nebeneinander innerhalb eines einzigen Hohlmantels angeordnet sind, welcher aus zwei konzentrischen Ringen (1, 2) und zwei seitlichen Flanschen (3) besteht und mit elektrisch isolierenden, fliessfähigen Kühlmitteln, sowie mit einer inneren Bleibeschichtung ausgestattet ist und ferner jeweils einer Röhre (4) gegenüberliegende Fenster (6) aufweist, und dass die Detektoren (7) zwischen den Fenstern nebeneinander am inneren Ring (1) angeordnet sind.

2. Tomographisches Gerät nach Anspruch 1, dadurch gekennzeichnet, dass die die Röntgenstrahlenbündel erzeugenden Röhren (4) je eine Kathode (23) aufweisende, mit einer Polarisationsvorrichtung (24) zusammenwirkende und mit einer feststehenden, geneigten Anodenfläche (22) versehene Röhren sind, die in Verbindung mit den Kollimationsmitteln (12) ein flaches und weit gespreiztes, fächerartig gebildetes Röntgenstrahlenbündel erzeugen.

3. Tomographisches Gerät nach einem der Ansprüche 1 oder 2, dadurch gekennzeichnet, dass alle Röhren (4) innerhalb des Mantels mit gemeinsamen Anschluss-Schienen (9, 10) zur Speisung mit Hochspannung, sowie mit Auslösevorrichtungen (11) und mit einem einzigen Oelkühlkreislauf ( 13, 14, 16, 17, 18) zur Kühlung ihrer Anoden versehen sind.

4. Tomographisches Gerät nach einem der Ansprüche 1 bis 3, dadurch gekennzeichnet, dass alle Kathodenauslösevorrichtungen (11) über eine von der Hochspannung isolierte Vorrichtung, wie Lichtleiter (37), mit Schaltkreisen (20-1,..., 20-n) verbunden sind, die ihrerseits an einen Steuerstromkreis (21) angeschlossen sind, derart, dass ein sehr schnelles selektives Auslösen der Röhren, beispielsweise einer Röhre nach der anderen, ermöglicht wird.

5. Tomographisches Gerät nach einem der Ansprüche 1 bis 4, dadurch gekennzeichnet, dass der Mantel um den Mittelpunkt des Kreises, auf dem die Röhren (4) angeordnet sind, innerhalb der Grenzen eines im Mittelpunkt gemessenen Winkelabstand je zweier benachbarter Röhren entsprechen Drehwinkels drehbar ist.

# Fig_1

0 005 666

Fig_2

Fig_3

Fig_4

Fig_5